# EUROPEAN PATENT SPECIFICATION

(11) **EP 2 898 824 B1**
(45) Date of publication and mention of the grant of the patent: **27.04.2016**
(21) Application number: 15150770.4
(22) Date of filing: 12.01.2015
(51) Int. Cl.: A61B 5/0215

(54) **Blood pressure measuring system**
Blutdruckmesssystem
Dispositif de mesure de pression artérielle

(30) Priority: 22.01.2014 JP 2014009623
(43) Date of publication of application: 29.07.2015
(73) Proprietor: Nihon Kohden Corporation, Shinjuku-ku Tokyo 161-8560 (JP)
(72) Inventor: Kubo, Hiroshi, Tokyo, 161-8560 (JP)
(74) Representative: Hoffmann Eitle

(56) References cited:
- US-A1- 2007 027 393
- US-A1- 2011 118 613
- US-B2- 7 862 513

## Description

### BACKGROUND

The presently disclosed subject matter relates to a system for invasively measuring the blood pressure value of the subject.

There are invasive blood pressure measuring methods in which a catheter or the like is inserted into the blood vessel of the subject to continuously measure the time varying blood pressure (for example, see JP-A-4-269938). The pressure generated in the blood vessel is converted to an electrical signal by a transducer which is connected to the catheter via a monitoring line. A sphygmomanometer displays the blood pressure value and waveform corresponding to the electrical signal, to a medical person or the like.

In an invasive blood pressure measurement, it is necessary to perform a process called "zero point calibration" for setting a reference value of the measurement. The zero point calibration is performed in the following manner. The transducer is placed in a reference point. When a pressure acting on the transducer is made zero (when the catheter is opened to the atmosphere), the electrical signal indicative of the blood pressure value is set to have a measurement reference value (for example, 0 V).

The zero point calibration is performed each time the subject is changed. Even during a measurement on the same subject, however, the measurement reference value is caused to vary (drift) by various causes. The largest variation cause is that the position relationship (relative altitude) of the blood pressure measurement position and the transducer is changed by a postural change of the subject or the like. When the measurement reference value varies, the blood pressure measurement is not correctly conducted, and therefore the zero point calibration must be again performed. In the zero point calibration, a three-way stopcock to which the transducer is connected must be opened to the atmosphere. A large burden is imposed to both the subj ect and the medical person by performing the zero point calibration each time the posture of the subject is changed.

### SUMMARY

The presently disclosed subject matter may provide a blood pressure measuring system in which the burden which is imposed to the subject and a medical person is reduced in an invasive blood pressure measurement.

The blood pressure measuring system may comprise: a sphygmomanometer which is configured to invasively measure a blood pressure value of a subject; a calibrating section which is configured to calibrate a measurement reference value of the blood pressure value; a first atmospheric pressure sensor which is adapted to be attached to the subject; a second atmospheric pressure sensor which is fixed to a predetermined position; a difference acquiring section which is configured to acquire a difference between a first atmospheric pressure value detected by the first atmospheric pressure sensor, and a second atmospheric pressure value detected by the second atmospheric pressure sensor; and a determining section which, based on the difference, is configured to determine whether a calibration of the measurement reference value is necessary or not.

When the determining section determines that the calibration is necessary, the calibrating section may automatically calibrate the measurement reference value.

The blood pressure measuring system may further comprise: a notifying section which, when the determining section determines that the calibration is necessary, is configured to output an alarm.

The blood pressure measuring system may further comprise : a temperature sensor which is disposed in adjacent to at least one of the first atmospheric pressure sensor and the second atmospheric pressure sensor. Based on the difference and a temperature detected by the temperature sensor, the determining section may determine whether the calibration is necessary or not.

The first atmospheric pressure sensor may be adapted to be attached to a chest of the subject.

The determining section may determine that the calibration of the measurement reference value is necessary when a value of the difference between the first and second atmospheric pressure values is varied at a degree which is larger than a predetermined value.

The calibrating section may store a relationship between a correction amount of the measurement reference value and a value of the difference between the first and second atmospheric pressure values.

### BRIEF DESCRIPTION OF THE DRAWINGS

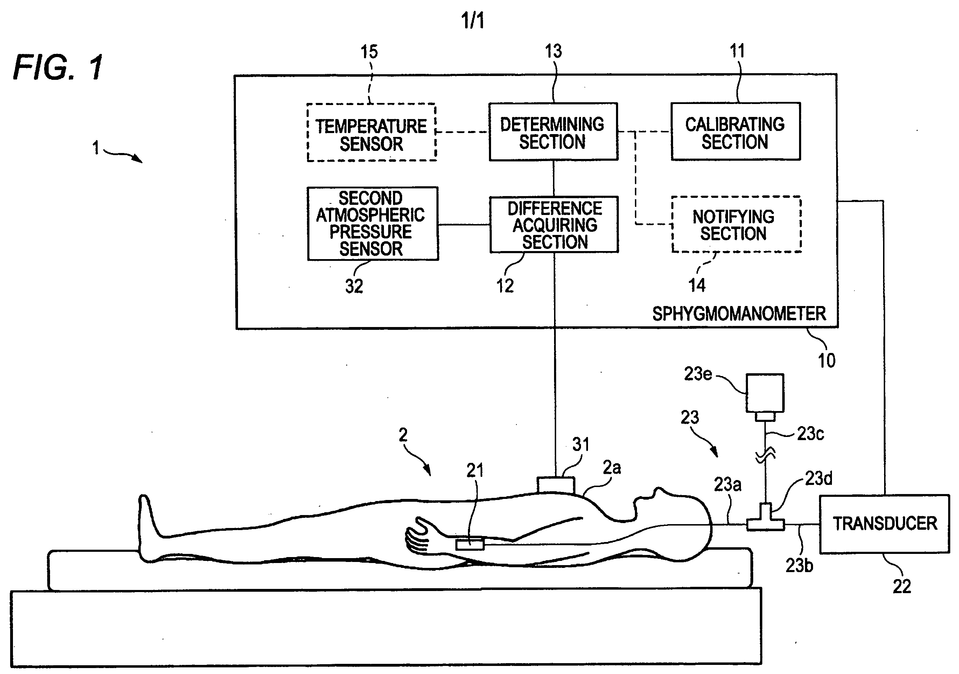

Fig. 1 is a view schematically showing a blood pressure measuring system of an embodiment of the presently disclosed subject matter.

### DETAILED DESCRIPTION OF EXEMPLARY EMBODIMENTS

An embodiment of the presently disclosed subject matter will be described in detail with reference to the accompanying drawing. Fig. 1 is a view schematically showing a blood pressure measuring system 1 of the embodiment of the presently disclosed subject matter. In the figure, the scale is adequately changed in order to draw components in a recognizable size.

The blood pressure measuring system 1 includes a sphygmomanometer 10. The sphygmomanometer 10 invasively measures the blood pressure value of the subject 2.

The illustrated embodiment shows the case where the arterial blood pressure is measured. A catheter (arterial needle) 21 is inserted into the radial artery of the subject 2. In the case where the venous blood pressure is measured, a Swan-Ganz catheter may be used. A transducer 22 is fixed at the level of the heart (the level corresponding to a half of the chest depth) of the subject 2. The catheter 21 and the transducer 22 are connected to each other through a monitoring line 23.

The monitoring line 23 includes a first tube 23a, a second tube 23b, a third tube 23c, a three-way stopcock 23d, and an infusion bottle 23e. The first tube 23a connects the catheter 21 to the three-way stopcock 23d. The second tube 23b connects the transducer 22 to the three-way stopcock 23d. The third tube 23c connects the infusion bottle 23e to the three-way stopcock 23d. The infusion bottle 23e stores a physiological saline solution containing heparin. When the three-way stopcock 23d is opened in all the directions, the first tube 23a, the second tube 23b, and the third tube 23c are filled with the physiological saline solution containing heparin.

The transducer 22 outputs an electrical signal corresponding to the pressure in the blood vessel of the subject 2, the pressure being transmitted through the physiological saline solution containing heparin. The sphygmomanometer 10 displays the blood pressure value and waveform corresponding to the electrical signal, to the user such as a medical person.

The blood pressure measuring system 1 includes a calibrating section 11. The calibrating section 11 is configured so as to calibrate the measurement reference value of the blood pressure value measured by the sphygmomanometer 10. When the measurement of the blood pressure is to be started, an initial zero point calibration of the sphygmomanometer 10 is performed. Specifically, the catheter 21 is set to be opened to the atmosphere to make the pressure acting on the transducer 22 zero. In this state, the calibrating section 11 performs a calibration so that the electrical signal supplied to the sphygmomanometer 10 has a reference value (for example, 0 V).

The blood pressure measuring system 1 includes a first atmospheric pressure sensor 31. The first atmospheric pressure sensor 31 is configured so as to be attachable to the subject 2. The first atmospheric pressure sensor 31 detects an atmospheric pressure (first atmospheric pressure value) at the attached position, and outputs a signal corresponding to the first atmospheric pressure value.

The blood pressure measuring system 1 includes a second atmospheric pressure sensor 32. The second atmospheric pressure sensor 32 is fixed to a predetermined position. The second atmospheric pressure sensor 32 detects an atmospheric pressure (second atmospheric pressure value) at the predetermined position, and outputs a signal corresponding to the second atmospheric pressure value.

The blood pressure measuring system 1 includes a difference acquiring section 12. The difference acquiring section 12 is configured so as to acquire the difference between the first atmospheric pressure value detected by the first atmospheric pressure sensor 31, and the second atmospheric pressure value detected by the second atmospheric pressure sensor 32.

The blood pressure measuring system 1 includes a determining section 13. Based on the difference acquired by the difference acquiring section 12, the determining section 13 determines whether a calibration of the measurement reference value of the sphygmomanometer 10 is necessary or not. In the case where, at the start of the measurement, the value of the difference between the first atmospheric pressure value and the second atmospheric pressure value is varied at a degree which is larger than a predetermined value, for example, it is determined that a calibration is necessary. The predetermined value is set to a value of a degree by which the execution of a correct measurement of the blood pressure value is impeded. In other words, even in the case where a postural change of the subject 2 occurs, when the change of the difference value is smaller than the predetermined value, the determining section 13 does not determine that a calibration is necessary.

In the case where a single atmospheric pressure sensor is used, it is impossible to determine from only a detection value of the atmospheric pressure during a measurement whether a change of the detection value is caused by that of the atmospheric pressure, or by a postural change of the subject. According to the configuration of the embodiment, by contrast, the difference between the first atmospheric pressure value detected by the first atmospheric pressure sensor 31 which is attached to the subject 2, and the second atmospheric pressure value detected by the second atmospheric pressure sensor 32 which is positionally fixed is monitored, and hence it is possible to grasp only the variation of the measurement reference value caused by a postural change of the subject 2. Therefore, an appropriate response is possible. When the degree of the postural change is within the range where the blood pressure measurement is not impeded, it is possible to determine that the calibration is not necessary. Consequently, the opportunity to perform the zero point calibration involving an operation of opening the three-way stopcock 23d to the atmosphere can be reduced, and the burden which is imposed to the subject and the medical person in the invasive blood pressure measurement can be reduced.

The calibrating section 11 may be configured so as to execute a calibration of the measurement reference value through a dial or switch which can be manually operated by a user. As indicated by the broken line in Fig. 1, alternatively, the calibrating section 11 and the determining section 13 may be communicably connected to each other. In the alternative, the determining section 13 supplies a signal indicative of the necessity/unnecessity of a calibration of the measurement reference value, to the calibrating section 11. If the determining section 13 determines that a calibration is necessary, the calibrating section 11 automatically sets the measurement reference value. For example, relationships between the correction amount of the measurement reference value, and the difference value between the first and second atmospheric pressure values which is acquired by the difference acquiring section 12, are stored in the calibrating section 11 in the form of a table or a function. If the determining section 13 determines that a calibration is necessary, the calibrating section 11 refers the difference value acquired by the difference acquiring section 12, and determines the correction amount of the measurement reference value based on the table or the function.

According to the configuration, the calibration of the measurement reference value is automated, and the necessity to perform the zero point calibration involving an operation of opening the three-way stopcock 23d to the atmosphere can be further suppressed. Therefore, the burden which is imposed to the subject and the medical person in the invasive blood pressure measurement can be further reduced.

As indicated by the broken lines in Fig. 1, the blood pressure measuring system 1 may include a notifying section 14. In this case, the notifying section 14 is configured so as to output an alarm when the determining section 13 determines that a calibration is necessary.

According to the configuration, in the case where the calibration of the measurement reference value is to be manually performed, the necessity can be notified more surely to the medical person. In the case where the calibration of the measurement reference value is to be automatically performed by the calibrating section 11, the medical person can easily grasp the situation where the calibration process is performed (i.e., the system is not in the usual measuring state). Therefore, the burden which is imposed to the medical person in an invasive blood pressure measurement can be further reduced.

As indicated by the broken lines in Fig. 1, the blood pressure measuring system 1 may include a temperature sensor 15. In this case, the temperature sensor 15 is disposed in adjacent to at least one of the first atmospheric pressure sensor 31 and the second atmospheric pressure sensor 32. In the example shown in Fig. 1, the temperature sensor 15 is disposed in the vicinity of the second atmospheric pressure sensor 32. In addition to or in place of this, the temperature sensor 15 may be disposed in the vicinity of the first atmospheric pressure sensor 31. The determining section 13 in the example determines whether a calibration of the measurement reference value is necessary or not, based on the difference value between the first and second atmospheric pressure values which is acquired by the difference acquiring section 12, and the temperature detected by the temperature sensor 15.

The measurement reference value of the sphygmomanometer 10 is varied also by a temperature change during a measurement. The result of the detection by the temperature sensor 15 can suggest the possibility that the measurement reference value is varied by a temperature change. According to the configuration, based also on an ambient temperature change during a measurement, it is determined whether the calibration is necessary or not. Hence, it is possible to provide a more correct determination result. Therefore, the burden which is imposed to the subject and the medical person in the invasive blood pressure measurement can be further reduced.

The attachment position of the first atmospheric pressure sensor 31 is not particularly limited as far as the difference acquiring section 12 can acquire the difference between the first and second atmospheric pressure values, and, based on the difference, the determining section 13 can determine whether the calibration is necessary or not. However, it is preferable that, as shown in Fig. 1, the first atmospheric pressure sensor 31 is placed on the chest 2a of the subject 2. This is because the transducer 22 is fixed at the level of the heart of the subject 2, and hence it is preferable that the first atmospheric pressure value detected by the first atmospheric pressure sensor 31 is an atmospheric pressure value in the vicinity of the heart of the subject 2.

According to the configuration, the difference between the- first and second atmospheric pressure values which is acquired by the difference acquiring section 12 further conforms to the spirit of the invention, and the correctness of the determination on the necessity/unnecessity of a calibration is enhanced. Therefore, the burden which is imposed to the subject and the medical person in the invasive blood pressure measurement can be further reduced.

The embodiment has been described in order to facilitate understanding of the invention, and is not intended to limit the invention. It is a matter of course that the invention may be changed or improved without departing the spirit thereof, and includes equivalent of the embodiment.

In the example shown in Fig. 1, the calibrating section 11, the difference acquiring section 12, the determining section 13, and the notifying section 14 are disposed in the sphygmomanometer 10. However, at least one of these sections may be disposed in an apparatus other than the sphygmomanometer 10.

It is not always required that the sphygmomanometer 10 is an independent apparatus. The sphygmomanometer may be provided as one function realized in a biological information monitoring apparatus which acquires and displays biological information such as an electrocardiogram.

According to an aspect of the presently disclosed subject matter, there is provided a blood pressure measuring system comprising: a sphygmomanometer which is configured to invasively measure a blood pressure value of a subject; a calibrating section which is configured to calibrate a measurement reference value of the blood pressure value; a first atmospheric pressure sensor which is adapted to be attached to the subject; a second atmospheric pressure sensor which is fixed to a predetermined position; a difference acquiring section which is configured to acquire a difference between a first atmospheric pressure value detected by the first atmospheric pressure sensor, and a second atmospheric pressure value detected by the second atmospheric pressure sensor; and a determining section which, based on the difference, is configured to determine whether a calibration of the measurement reference value is necessary or not. According to the configuration, the difference between the first atmospheric pressure value detected by the first atmospheric pressure sensor which is attached to the subject, and the second atmospheric pressure value detected by the second atmospheric pressure sensor which is positionally fixed is monitored. Therefore, it is possible to grasp only the variation of the measurement reference value caused by a postural change of the subject. Therefore, an appropriate response is possible. When the degree of the postural change is within the range where the blood pressure measurement is not impeded, it is possible to determine that the calibration is not necessary. Consequently, the opportunity to perform the zero point calibration involving an operation of opening a three-way stopcock to the atmosphere can be reduced, and the burden which is imposed to the subject and a medical person in an invasive blood pressure measurement can be reduced.

When the determining section determines that the calibration is necessary, the calibrating section may automatically calibrate the measurement reference value. According to the configuration, the calibration of the measurement reference value is automated, and the necessity to perform the zero point calibration involving an operation of opening the three-way stopcock to the atmosphere can be further suppressed. Therefore, the burden which is imposed to the subject and a medical person in an invasive blood pressure measurement can be further reduced.

The blood pressure measuring system may further comprise: a notifying section which, when the determining section determines that the calibration is necessary, is configured to output an alarm. According to the configuration, in the case where the calibration of the measurement reference value is to be manually performed, the necessity can be notified more surely to a medical person. In the case where the calibration of the measurement reference value is to be automatically performed by the calibrating section, a medical person can easily grasp the situation where the calibration process is performed (i.e., the system is not in the usual measuring state). Therefore, the burden which is imposed to the medical person in an invasive blood pressure measurement can be further reduced.

The blood pressure measuring system may further comprise: a temperature sensor which is disposed in adjacent to at least one of the first atmospheric pressure sensor and the second atmospheric pressure sensor, and, based on the difference and a temperature detected by the temperature sensor, the determining section may determine whether the calibration is necessary or not. The measurement reference value of a sphygmomanometer is varied also by a temperature change during a measurement. According to the configuration, based also on an ambient temperature change during a measurement, it is determined whether the calibration is necessary or not. Hence, it is possible to provide a more correct determination result. Therefore, the burden which is imposed to the subject and a medical person in an invasive blood pressure measurement can be further reduced.

The first atmospheric pressure sensor may be adapted to be attached to a chest of the subject. According to the configuration, the difference between the first and second atmospheric pressure values which is acquired by the difference acquiring section further conforms to the scope of the invention, and the correctness of the determination on the necessity/unnecessity of a calibration is enhanced. Therefore, the burden which is imposed to the subject and a medical person in an invasive blood pressure measurement can be further reduced.

## Claims

1. A blood pressure measuring system comprising:
a sphygmomanometer (10) which is configured to invasively measure a blood pressure value of a subject;
a calibrating section (11) which is configured to calibrate a measurement reference value of the blood pressure value; **characterised by**
a first atmospheric pressure sensor (31) which is adapted to be attached to the subject;
a second atmospheric pressure sensor (32) which is fixed to a predetermined position;
a difference acquiring section (12) which is configured to acquire a difference between a first atmospheric pressure value detected by the first atmospheric pressure sensor (31), and a second atmospheric pressure value detected by the second atmospheric pressure sensor (32); and
a determining section (13) which, based on the difference, is configured to determine whether a calibration of the measurement reference value is necessary or not.

2. The blood pressure measuring system according to claim 1, wherein, when the determining section (13) determines that the calibration is necessary, the calibrating section (11) automatically calibrates the measurement reference value.

3. The blood pressure measuring system according to claim 1 or 2, further comprising: a notifying section (14) which, when the determining section (13) determines that the calibration is necessary, is configured to output an alarm.

4. The blood pressure measuring system according to any one of claims 1-3, further comprising: a temperature sensor (15) which is disposed in adjacent to at least one of the first atmospheric pressure sensor (31) and the second atmospheric pressure sensor (32), wherein,
based on the difference and a temperature detected by the temperature sensor (15), the determining section (13) determines whether the calibration is necessary or not.

5. The blood pressure measuring system according to any one of claims 1-4, wherein the first atmospheric pressure sensor (31) is adapted to be attached to a chest of the subject.

6. The blood pressure measuring system according to any one of claims 1-5, wherein the determining section (13) determines that a calibration is necessary when the value of the difference between the first atmospheric pressure value and the second atmospheric pressure value is varied at a degree which is larger than a predetermined value.

7. The blood pressure measuring system according to any one of claims 1-6, the calibrating section (11) stores relationships between the difference and between the first and second atmospheric pressure value.

## Patentansprüche

1. Ein Blutdruckmesssystem, umfassend:
ein Sphygmomanometer (10), welches ausgebildet ist zum invasiven Messen eines Blutdruckwerts einer Testperson;
eine Kalibriereinheit (11), welche ausgebildet ist zum Kalibrieren eines Messreferenzwerts des Blutdruckwerts; **gekennzeichnet durch**
einen ersten Atmosphärendruck-Sensor (31), welcher angepasst ist, an der Testperson angebracht zu werden;
einen zweiten Atmosphärendruck-Sensor (32), welcher an einer vorbestimmten Position befestigt ist;
eine Unterschiederfassungseinheit (12), welche ausgebildet ist zum Erfassen eines Unterschieds zwischen einem **durch** den ersten Atmosphärendruck-Sensor (31) detektierten ersten Atmosphärendruckwert und einem **durch** den zweiten Atmosphärendruck-Sensor (32) detektierten zweiten Atmosphärendruckwert; und
eine Bestimmungseinheit (13), welche basierend auf dem Unterschied ausgebildet ist, zu bestimmen, ob eine Kalibration des Messreferenzwerts notwendig ist oder nicht.

2. Blutdruckmesssystem gemäß Anspruch 1, wobei, wenn die Bestimmungseinheit (13) bestimmt, dass die Kalibration notwendig ist, die Kalkulationseinheit (11) den Messreferenzwert automatisch kalibriert.

3. Blutdruckmesssystem gemäß Anspruch 1 oder 2, weiter umfassend: eine Benachrichtigungseinheit (14), welche, wenn die Bestimmungseinheit (13) bestimmt, dass die Kalibration notwendig ist, ausgebildet ist einen Alarm auszugeben.

4. Blutdruckmesssystem gemäß einem der Ansprüche 1-3, weiter umfassend: einen Temperatursensor (15), welcher benachbart zu dem ersten Atmosphärendruck-Sensor (31) und/oder dem zweiten Atmosphärendruck-Sensor (32) angeordnet ist, wobei
basierend auf dem Unterschied und einer durch den Temperatursensor (15) detektierten Temperatur die Bestimmungseinheit (13) bestimmt, ob die Kalibration notwendig ist oder nicht.

5. Blutdruckmesssystem gemäß einem der Ansprüche 1-4, wobei der erste Atmosphärendruck-Sensor (31) angepasst ist, an einer Brust der Testperson angebracht zu werden.

6. Blutdruckmesssystem gemäß einem Ansprüche 1-5, wobei die Bestimmungseinheit (13) bestimmt, dass eine Kalibration notwendig ist, wenn der Wert des Unterschieds zwischen dem ersten Atmosphärendruckwert und dem zweiten Atmosphärendruckwert sich um einen Grad verändert, welcher größer als ein vorbestimmter Wert ist.

7. Blutdruckmesssystem gemäß einem der Ansprüche 1-6, wobei die Kalibriereinheit (11) Beziehungen zwischen den Unterschieden zwischen dem ersten und zweiten Atmosphärendruckwert speichert.

## Revendications

1. Système de mesure de la pression artérielle comprenant :
un sphygmomanomètre (10) qui est configuré pour mesurer de manière invasive une valeur de la pression artérielle d'un sujet ;
une section d'étalonnage (11) qui est configurée pour étalonner une valeur de référence de mesure de la valeur de la pression artérielle ;
**caractérisé par** :
un premier capteur de pression atmosphérique (31) qui est adapté pour être attaché au sujet ;
un second capteur de pression atmosphérique (32) qui est fixé en position prédéterminée ;
une section d'acquisition de différence (12) qui est configurée pour acquérir une différence entre une première valeur de la pression atmosphérique détectée par le premier capteur de pression atmosphérique (31) et une seconde valeur de la pression atmosphérique détectée par le second capteur de pression atmosphérique (32) ; et
une section de détermination (13) qui, sur la base de la différence, est configurée pour déterminer si un étalonnage de la valeur de référence de mesure est nécessaire ou non.

2. Système de mesure de la pression artérielle selon la revendication 1, dans lequel, quand la section de détermination (13) détermine que l'étalonnage est nécessaire, la section d'étalonnage (11) étalonne automatiquement la valeur de référence de mesure.

3. Système de mesure de la pression artérielle selon la revendication 1 ou 2, comprenant en outre une section de notification (14) qui, quand la section de détermination (13) détermine que l'étalonnage est nécessaire, est configurée pour délivrer une alarme.

4. Système de mesure de la pression artérielle selon l'une quelconque des revendications 1 à 3, comprenant en outre un capteur de température (15) qui est disposé de manière adjacente à au moins l'un du premier capteur de pression atmosphérique (31) et du second capteur de pression atmosphérique (32), dans lequel,
sur la base de la différence et d'une température détectée par le capteur de température (15), la section de détermination (13) détermine si l'étalonnage est nécessaire ou non.

5. Système de mesure de la pression artérielle selon l'une quelconque des revendications 1 à 4, dans lequel le premier capteur de pression atmosphérique (31) est adapté pour être attaché à la poitrine du sujet .

6. Système de mesure de la pression artérielle selon l'une quelconque des revendications 1 à 5, dans lequel la section de détermination (13) détermine qu'un étalonnage est nécessaire quand la valeur de la différence entre la première valeur de la pression atmosphérique et la seconde valeur de la pression atmosphérique varie à un degré qui est plus grand qu'une valeur prédéterminée.

7. Système de mesure de la pression artérielle selon l'une quelconque des revendications 1 à 6, dans lequel la section d'étalonnage (11) stocke des relations entre la différence et entre les première et seconde valeurs de la pression atmosphérique.
